# EUROPÄISCHE PATENTSCHRIFT

(11) **EP 1 011 534 B1**
(45) Veröffentlichungstag und Bekanntmachung des Hinweises auf die Patenterteilung: **26.11.2003**
(21) Anmeldenummer: 98954257.6
(22) Anmeldetag: 05.09.1998
(51) Int. Cl.: A61F 2/06

(54) **STENT ZUR TRANSLUMINALEN IMPLANTATION**
STENT FOR TRANSLUMINAL IMPLANTATION
EXTENSEUR POUR IMPLANTATION TRANSLUMINALE

(30) Priorität: 09.09.1997 DE 29716117 U
(43) Veröffentlichungstag der Anmeldung: 28.06.2000
(73) Patentinhaber: Micro Science Medical AG, 75443 Ötisheim (DE)
(72) Erfinder: STARCK, Bernd, D-75443 Ötisheim (DE); ALTER, Robert, D-85579 Neubiberg (DE)
(74) Vertreter: Reinhardt, Harry
(86) Internationale Anmeldenummer: EP9805651
(87) Internationale Veröffentlichungsnummer: WO99012495

(56) Entgegenhaltungen:
- WO-A-95/31945
- DE-U- 29 701 758
- DE-U- 29 702 671

## Beschreibung

Die Erfindung betrifft einen Stent zur transluminalen Implantation in Hohlorgane, insbesondere in Blutgefäße nach dem Oberbegriff des Anspruchs 1.

Ein derartiger Stent ist aus EP 734 698 A2 bekannt und wird zur Rekanalisation von krankhaft veränderten Hohlorganen wie z.B. Blutgefäßen eingesetzt. Der Stent wird im komprimierten Zustand über einen Einführkatheder an die zu behandelnde Stelle innerhalb des Hohlorgans eingeführt und dort z.B. durch einen speziellen Ballonkatheder expandiert. Der Stent bleibt dann im expandierten Zustand stehen, so daß die Gefäßwandung so abgestützt ist, daß sich im wesentlichen die ursprüngliche Form des Gefäßes wieder einstellt. Während der Stent im komprimierten Zustand möglichst klein sein soll, um ihn leicht an die zu behandelnde Stelle überführen zu können, soll er im Gegensatz dazu im expandierten Zustand die infolge der Expansion des Gefäßes auftretenden Kräfte zuverlässig übertragen können und dennoch möglichst flexibel sein, was an und für sich gegenläufige Voraussetzungen für den Stent sind. Um die Längenverkürzung bei der Expansion des Stents auszugleichen, die deswegen unerwünscht ist, da der Stent ansonsten nicht genau positioniert werden kann, werden bereits Zwischenelemente vorgeschlagen, die in Längsrichtung plastisch verformt werden, so daß die in Umfangsrichtung angeordneten Dehnelemente des Stents möglichst im komprimierten und expandierten Zustand an der gleichen Stelle zu liegen kommen. Diese Zwischenelemente sind V-förmig oder schleifenförmig, so daß eine entsprechende Längendehnung unter Streckung des "V" oder der Schleife möglich ist.

Dennoch reichen diese Maßnahmen nicht aus, eine Längenverkürzung weitestgehend zu verringern und die in radialer Richtung des Stents gedehnten Dehnelemente besitzen noch einen Recoil-Effekt und neigen damit zum Zurückfedern in ihre Ausgangsposition, so daß es trotz dieser Maßnahmen innerhalb der ersten 6 Monate nach der Operation in einem Drittel aller Fälle zu einem Wieder-Verschluß kommt.

Aus DE 295 21 206 U1 ist ein Stent bekannt, in den zwei Mäandermuster miteinander verknüpft sind. Das eine Mäandermuster ist in Längsrichtung des Stents angeordnet, das zweite Mäandermuster in Umfangsrichtung. Zwischen den einzelnen Mustern sind Schlaufen vorgesehen, die als Zwischenelemente bestimmt sind, um die einzelnen Mäandermuster voneinander zu entkoppeln. Auch hier besteht die Gefahr eines Recoil-Effektes, da zwar die Schlaufen die Muster entkoppeln und insofern eine Verkürzungskompensation teilweise verwirklichen, die verbleibenden Muster jedoch nicht über den elastischen Zustand hinaus gedehnt werden. Aufgrund der Materialmenge muß bei der Expansion des Stents zudem mit entsprechenden Drücken gearbeitet werden, so daß es zu einer Verletzung der Gefäße kommen kann.

Aus DE 43 03 181 A1 ist ein Stent bekannt, der über den Umfang sich erstreckende Mäanderbahnen aufweist, die untereinander nur stellenweise unmittelbar verbunden sind, um eine entsprechende Flexibilität sicherzustellen. Da keine Zwischenelemente vorgesehen sind, neigt dieser Stent zur Längenverkürzung bei Expansion. Da nicht gezielt eine plastische Verformung auch von den einzelnen Mäanderbahnen vorgenommen wird, tritt auch hier ein Rückfederungseffekt ein. Durch die gleichmäßige Verteilung der Mäanderbahnen wird bei diesem Stent wie auch bei den vorausgegangenen ein sogenannter "Trompeteneffekt" erzielt. Dabei dehnen sich die Stent-Enden weiter auf als das mittlere Segment, so daß der Stent mit seinen Enden sich in der Gefäßwandung einhakt. Wenn er sich gleichzeitig verkürzt, kann es zu Verletzungen der Gefäßwandung kommen, die eventuell über den Selbstheil-Effekt der Gefäße hinausgehen.

Ausgehend von diesem Stand der Technik liegt der vorliegenden Erfindung die Aufgabe zugrunde, einen Stent der eingangs genannten Gattung derart weiterzubilden, daß eine Längenverkürzung beim Aufdehnen vermindert ist.

Diese Aufgabe wird durch einem Stent mit den Merkmalen des Anspruchs 1 gelöst.

Bei diesem Stent sind die im Stand der Technik bekannten Zwischenelemente vorgesehen, die an ersten Dehnelementen angreifen. Zwischen diesen ersten Dehnelementen ist jedoch wenigstens ein weiteres Dehnelement vorgesehen, das über eine höhere elastische Verformbarkeit verfügt als das erste Dehnelement. Dadurch stellt sich eine höhere plastische Verformung der ersten Dehnelemente ein, so daß es in Verbindung mit den Zwischenelementen zu einer geringeren Längenverkürzung kommt. Die umfangsseitige Expansion wird aber gleichzeitig durch die elastischeren weiteren Dehnelemente gewährleistet, so daß diese nicht vollständig bis in den plastischen Bereich verformt werden müssen und insofern über entsprechende Stützkräfte verfügen, um die Gefäßwandung zuverlässig abzustützen. Dadurch ist in Umfangsrichtung keine oder eine nur sehr geringe Überdehnung erforderlich, so daß der Recoil-Effekt verringert ist. Dies führt zu einer Reduzierung von Komplikationen, Re-Eingriffen und Re-Verschlüssen, so daß dieser Stent insgesamt gesehen äußerst wirtschaftlich ist.

Kurzbeschreibung der Figuren
- Fig. 1: Eine Abwicklung eines komprimierten Stents,
- Fig. 2: eine Abwicklung des Stents nach seiner Herstellung,
- Fig. 3: eine Abwicklung des Stents im expandierten Zustand.

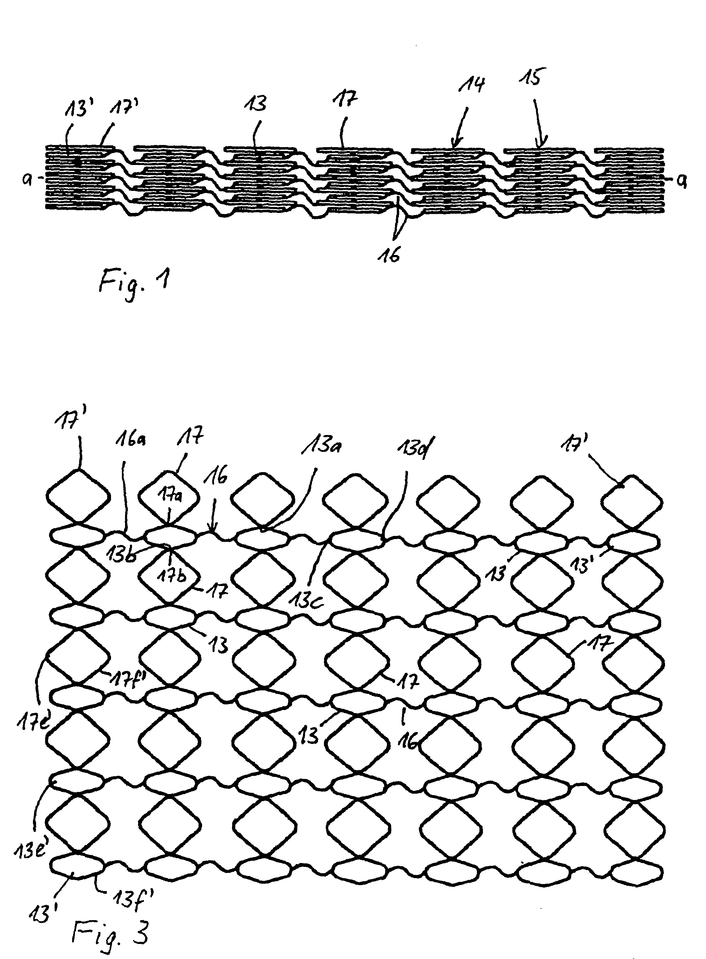

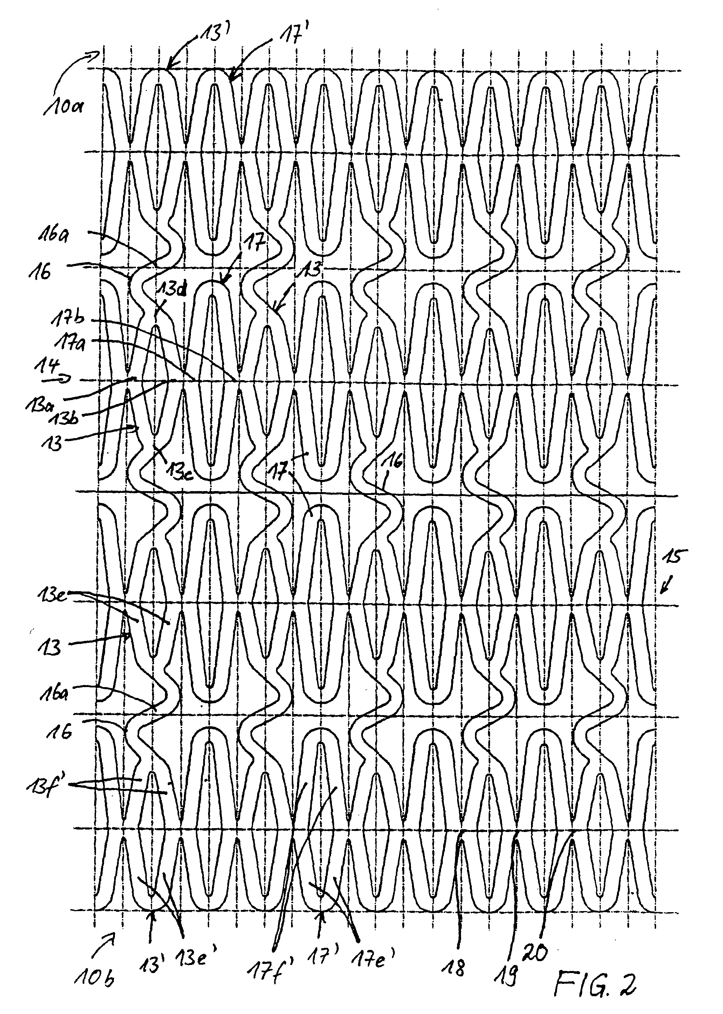

Der Stent ist zur transluminalen Implantation in Hohlorgane, insbesondere in Blutgefäße bestimmt. Gemäß Fig. 1 besitzt er einen im wesentlichen röhrenförmigen Körper, der aus einem komprimierten Zustand mit einem ersten Querschnitt t in einen expandierten Zustand mit vergrößertem Querschnitt 12 gemäß Fig. 3 überführbar ist. Zur Verdeutlichung zeigen die Figuren die jeweiligen Abwicklungen. Nach der Herstellung besitzt der Stent eine Form gemäß Fig. 2, wird beim Einführen in den Körper komprimiert, so daß sich eine Abwicklung gemäß Fig. 1 ergibt, und am Einsatzort expandiert (Fig. 3).

Gemäß Fig. 2 besitzt der Stent mehrere in Umfangsrichtung miteinander verbundene erste Dehnelemente 13 als auch weitere Dehnelemente 17. Diese Dehnelemente bilden wenigstens zwei Ketten 14,15, die in Fig. 2 durch zwei Abschlußketten ergänzt sind, auf deren Ausgestaltung noch einzugehen ist. Der Stent ist hier nach einem regelmäßigen Raster aufgebaut, so daß er problemlos abschnittsweise je nach Einsatzzweck verlängert werden kann. Zwischen den Ketten 14,15 sind mehrere Zwischenelemente 16 vorgesehen. Diese Zwischenelemente besitzen wenigstens einen schräg zur Längsachse a-a des Stents angeordneten Abschnitt 16a und dienen zumindest teilweise zur Kompensation der Längenverkürzung bei Expansion des Stents. Bei der Expansion wird dabei das Zwischenelement 16 zunächst in Längsrichtung gestreckt und dann bedarfsweise plastisch verformt.

Die weiteren Dehnelemente 17, die zwischen den ersten Dehnelementen 13 vorgesehen sind, an denen die Zwischenelemente 16 angreifen, besitzen eine höhere elastische Verformbarkeit als die ersten Dehnelemente 13. Dadurch wird eine plastische Verformung mehr oder weniger bei den ersten Dehnelementen erzwungen, so daß auch dies mit dazu beiträgt, eine Längenverkürzung auszugleichen. Die dennoch erforderliche Expansion wird dabei funktionell den weiteren Dehnelementen in erster Linie zugewiesen, wenngleich, wie Fig. 3 verdeutlicht, auch die ersten Dehnelemente in Umfangsrichtung gedehnt werden.

Diese höhere elastische Verformbarkeit der weiteren Dehnelemente gegenüber den ersten Dehnelementen kann auf unterschiedliche Weise verwirklicht werden, wobei im Ausführungsbeispiel dies durch eine geometrische Gestaltung verwirklicht wird, indem nämlich die weiteren Dehnelemente 17 im komprimierten Zustand eine größere Länge in Richtung der Länsachse a-a als die ersten Dehnelemente aufweisen, wie insbesondere Fig. 2 verdeutlicht. Da an den ersten Dehnelementen gleichzeitig die Zwischenelemente angreifen, führt dies nach entsprechender Dehnung der Zwischenelemente zu einer erzwungenen plastischen Verformung auch der ersten Dehnelemente. Auch beim weiteren Komprimieren trägt die Rautenform dazu bei, daß der Stent leicht komprimiert werden kann. So entspricht die Länge eines ersten Dehnelements 13 zusammen mit dem Zwischenelement 16 etwa der Länge eines weiteren Dehnelements. Dies trägt dazu bei, daß der Stent sich über nahezu die gesamte Gefäßwand abstützen kann, so daß er im Materialquerschnitt verringert werden kann und sich ein äußerst filigraner Aufbau ergibt. Der filigrane Aufbau führt dazu, daß weniger Seitenäste durch zu große Stege verschlossen werden und sich dennoch der Stent bereits mit geringem Druck öffnen läßt, so daß der Stent bereits bei einem Druck von ca. 6 bar komplett geöffnet ist. Gleichwohl können durch die Abstützung der gesamten Gefäßwand die entsprechenden Kräfte zum Aufweiten des Gefäßes aufgebracht werden. Im expandierten Zustand gemäß Fig. 3 gleichen sich die Längen der ersten Dehnelemente 13 und der weiteren Dehnelemente 17 dann wieder in Längsrichtung a-a aneinander an.

Gemäß Fig. 2 sind im Ausführungsbeispiel die ersten Dehnelemente 13 als auch die weiteren Dehnelemente 17 rautenförmig, wobei der Abstand der einzelnen Dehnelemente zueinander gleichmäßig ist. Insofern sind die Verbindungspunkte 18,19,20 zwischen den einzelnen Dehnelementen 13,17 in Umfangsrichtung gleichmäßig beabstandet, so daß die Breite der Dehnelemente in Umfangsrichtung gleich ist. Die weiteren Dehnelemente 17 besitzen zwei in etwa in Umfangsrichtung des Stents gegenüberliegende Ecken 17a,17b, an denen sie mit den Ecken 13a,13b der ersten Dehnelemente 13 verbunden sind. Die anderen beiden Ecken der weiteren Dehnelemente 17 liegen frei. Die beiden weiteren Ecken 13c,13d der ersten Dehnelemente sind jedoch mit den Zwischenelementen 16 verbunden. Wenngleich es grundsätzlich möglich ist, zwischen den ersten Dehnelementen mehrere weitere Dehnelemente vorzusehen, wird im Ausführungsbeispiel eine wechselweise Anordnung von ersten Dehnelementen 13 und weiteren Dehnelementen 17 vorgesehen, um eine möglichst gleichmäßige Verformung zu erzielen. Die Anbindung führt zu der langgestreckten Form der ersten Dehnelemente 13 in Fig. 3.

Fig. 2 verdeutlicht bereits, daß die Ecken und Kanten sowohl der Dehnelemente 13,17 als auch der Zwischenelemente 16 abgerundet und strömungsoptimiert sind. Dies wird auch im Querschnitt fortgesetzt, um Komplikationen, Ablagerungen und Abwehrreaktionen des Körpers möglichst im Vorfeld entgegen zu wirken. Ein Verkanten der Stege wird vermieden und die dadurch hervorgerufene Verletzungsgefahr verringert. Die Strömungsoptimierung vermindert eine Schädigung von Blutkörperchen.

Betrachtet man das in Fig. 2 gestrichelt dargestellte Raster des Stents genauer, so stellt man fest, daß die Außenbereiche kürzer sind als die Innenbereiche. Dies wird vorgenommen, um in diesem Bereich weniger Material vorzusehen, das ansonsten bei der Verformung der Stent in seinen expandierten Zustand dazu neigen könnte, sich nach außen aufzudehnen ("Trompeteneffekt"). Insofern sind die an den Enden 10a,10b des Stents liegenden äußeren weiteren Dehnelemente 17' in Längsrichtung des Stents kürzer als die innen liegenden weiteren Dehnelemente 17. Eine genaue Betrachtung ergibt, daß insofern die äußere Hälfte 17e' des äußeren weiteren Dehnelements 17' kürzer ist als dessen innere Hälfte 17f'.

Im Gegenzug dazu sind die an den Enden liegenden äußeren ersten Dehnelemente 13', also die Dehnelemente, die mit den Zwischenelementen 16 gekoppelt sind, in Längsrichtung des Stents länger als die innen liegenden ersten Dehnelemente 13. Fig. 2 zeigt, daß die äußere Hälfte 13e' des äußeren ersten Dehnelements 13' länger ist als dessen innere Hälfte 13f'. Im Ausführungsbeispiel wird angestrebt, daß die äußeren Hälften 13e',17e' in ihrer Form einander entsprechend ausgebildet sind, so daß sich eine gleichmäßige umfangsseitige Verformung ergibt. Es ist zu vermuten, daß dies dazu führt, daß im Zusammenspiel der Verformung zwischen ersten und weiteren äußeren Dehnelementen 13',17' ein Aufweiten der Enden 10a,10b verringert ist. Selbst bei zu starker plastischer Verformung weiten die Enden des Stents sich weniger au fals im Stand der Technik bisher der Fall. Hier wird der gegenläufige Effekt eingesetzt, so daß selbst bei den ersten Dehnelementen eine größere Elastizität möglich ist als dies im Innenbereich der Fall ist, so daß es nicht zu einem Aufweiten der Endbereiche kommt. Durch die gleichmäßige Ausgestaltung der Enden aller Dehnelemente wird dieser Effekt unterstützt.

Während im Ausführungsbeispiel dieser Effekt lediglich bei den außen liegenden Dehnelementen 13',17' bewirkt wird, so ist es durchaus auch denkbar, das Raster von den Enden des Stents bis hin zur Mitte allmählich aufzuweiten, um hierdurch eine gleichmäßigere Verformung zu erzielen. Ebenso können andere Formen der Dehnelemente und der Zwischenelemente vorgesehen werden, sofern lediglich ein entsprechendes Zusammenspiel von plastischer und elastischer Verformung gewährleistet ist, das die Nachteile des Standes der Technik beseitigt.

## Patentansprüche

1. Stent zur transluminalen Implantation in Hohlorgane, insbesondere in Blutgefäße mit
- einem im wesentlichen röhrenförmigen Körper, der aus einem komprimierten Zustand mit einem ersten Querschnitt in einen expandierten Zustand mit vergrößertem Querschnitt überführbar ist,
- mehreren in Umfangsrichtung miteinander verbundenen ersten Dehnelementen (13), die wenigstens zwei Ketten (14,15) bilden,
- mehreren Zwischenelementen (16), die Dehnelemente (13) benachbarter Ketten (14,15) miteinander verbinden und die wenigstens einen schräg zur Längsachse (a-a) des Stents angeordneten Abschnitt (16a) zur zumindest teilweisen Kompensation der Längenverkürzung bei Expansion des Stents aufweisen,
**dadurch gekennzeichnet, daß** zwischen den ersten Dehnelementen (13) benachbarter Ketten (14,15) die durch Zwischenelemente (16) verbunden sind, wenigstens ein weiteres Dehnelement (17) vorgesehen ist, das eine höhere elastische Verformbarkeit aufweist als die ersten Dehnelemente (13).

2. Stent nach Anspruch 1, **dadurch gekennzeichnet, daß** die weiteren Dehnelemente (17) im komprimierten Zustand eine größere Länge in Richtung der Längsachse (a-a) des Stents aufweisen als die ersten Dehnelemente (13).

3. Stent nach Anspruch 1, **dadurch gekennzeichnet, daß** die ersten (13) und die weiteren Dehnelemente (17) im komprimierten Zustand rautenförmig sind, wobei die weiteren Dehnelemente (17) an zwei in etwa in Umfangsrichtung des Stents gegenüberliegenden Ecken (17a,17b) mit den ersten Dehnelementen (13) verbunden sind, die an ihren beiden anderen Ecken (13c,13d) ihrer Rautenform mit den Zwischenelementen (16) verbunden sind.

4. Stent nach Anspruch 1, **dadurch gekennzeichnet, daß** entlang des Umfangs des Stents wechselweise ein erstes (13) und ein weiteres Dehnelement (17) vorgesehen sind.

5. Stent nach Anspruch 1, **dadurch gekennzeichnet, daß** Ecken und Kanten der Dehnelemente (13,17) und/oder der Zwischenelemente (16) abgerundet und strömungsoptimiert sind.

6. Stent nach Anspruch 1, **dadurch gekennzeichnet, daß** am Ende (10a,10b) des Stents liegende äußere weitere Dehnelemente (17') in Längsrichtung des Stents kürzer sind als innen liegende weitere Dehnelemente (17).

7. Stent nach Anspruch 6, **dadurch gekennzeichnet, daß** die am Ende liegende äußere Hälfte (17e') des äußeren weiteren Dehnelements (17') kürzer ist als dessen innere Hälfte (17f').

8. Stent nach Anspruch 1, **dadurch gekennzeichnet, daß** am Ende (10a,10b) des Stents liegende äußere erste Dehnelemente (13') in Längsrichtung des Stents länger sind als innen liegende erste Dehnelemente (13).

9. Stent nach Anspruch 8, **dadurch gekennzeichnet, daß** die am Ende (10a,10b) liegende äußere Hälfte (13e') des äußeren ersten Dehnelements (13') länger ist als dessen innere Hälfte (13f').

10. Stent nach Anspruch 7, **dadurch gekennzeichnet, daß** am Ende (10a,10b) des Stents liegende äußere Hälften (13e') von äußeren ersten Dehnelementen (13') in ihrer Form den äußeren Hälften (17e') der äußeren weiteren Dehnelemente (17') entsprechen.

11. Stent nach Anspruch 1, **dadurch gekennzeichnet, daß** Verbindungspunkte (18,19,20) zwischen den Dehnelementen (13,17) in Umfangsrichtung gleichmäßig beabstandet sind.

## Claims

1. Stent for transluminal implantation in tubular organs, especially blood vessels, comprising
- an essentially tubular body which is modifiable from a compressed state with an initial diameter into an expanded state with an enlarged diameter,
- several first expansion elements (13) which are mutually connected in circumferential direction and form at least two chains (14, 15),
- several intermediate elements (16) which connect the expansion elements (13) of neighboring chains (14, 15) and incorporate at least one section (16a) that extends at an angle relative to the longitudinal axis (a-a) of the stent, so as to at least partially compensate for the reduction in length of the stent as it is expanded,
**characterized in that**, between the first expansion elements (13) of neighboring chains (14, 15) which are connected by intermediate elements (16), at least one additional expansion element (17) is provided the elastic deformability of which is greater than that of the first expansion elements (13).

2. Stent as in claim 1, **characterized in that**, in the compressed state, the additional expansion elements (17) are of greater length in the direction of the longitudinal axis (a-a) of the stent than the first expansion elements (13).

3. Stent as in claim 1, **characterized in that**, in the compressed state, the first (13) and the additional expansion elements (17) are rhombus-shaped, wherein the additional expansion elements (17) are connected at two corners (17a, 17b), mutually opposite in essentially the circumferential direction of the stent, to the first expansion elements (13) whose two other rhombus corners (13c, 13d) are connected to the intermediate elements (16).

4. Stent as in claim 1, **characterized in that** a first (13) and an additional expansion element (17) are positioned in alternating fashion along the circumference of the stent.

5. Stent as in claim 1, **characterized in that** corners and edges of the expansion elements (13, 17) and/or of the intermediate elements (16) are rounded and flow-oriented.

6. Stent as in claim 1, **characterized in that** the outer additional expansion elements (17') positioned at the respective end (10a, 10b) of the stent are shorter in the longitudinal direction of the stent than the inner additional expansion elements (17).

7. Stent as in claim 6, **characterized in that** the outer half (17e') of the outer additional expansion element (17') positioned at the end of the stent is shorter than its inner half (17f').

8. Stent as in claim 1, **characterized in that** the outer first expansion elements (13') positioned at the respective end (10a, 10b) of the stent are longer in the axial direction of the stent than the inner first expansion elements (13).

9. Stent as in claim 8, **characterized in that** the outer half (13e') of the outer first expansion element (13') positioned at the end of the stent is longer than its inner half (13f ).

10. Stent as in claim 7, **characterized in that** the outer halves (13e') of the outer first expansion elements (13') positioned at the respective end (10a, 10b) of the stent correspond in their shape to the outer halves (17e') of the outer additional expansion elements (17').

11. Stent as in claim 1, **characterized in that** the junctions (18, 19, 20) between the expansion elements (13, 17) are equidistant from one another in the circumferential direction.

## Revendications

1. Extenseur pour implantation transluminale dans des organes creux, en particulier des vaisseaux sanguins, comportant
- un corps de forme sensiblement tubulaire qui peut passer d'un état comprimé avec une première section transversale à un état expansé avec section transversale plus grande,
- plusieurs premiers éléments extensibles (13) reliés entre eux dans la direction périphérique, qui forment au moins deux chaînes (14, 15),
- plusieurs éléments intermédiaires (16) qui relient entre eux les éléments extensibles (13) de chaînes (14, 15) voisines et qui comportent au moins un tronçon (16a) disposé obliquement par rapport à l'axe longitudinal (a-a) de l'extenseur, pour compenser au moins en partie le raccourcissement de la longueur lors de l'expansion de l'extenseur,
**caractérisé en ce qu'**il est prévu, entre les premiers éléments extensibles (13) de chaînes (14, 15) voisines qui sont reliées par des éléments intermédiaires (16), au moins un autre élément extensible (17) qui présente une plus grande déformabilité élastique que les premiers éléments extensibles (13).

2. Extenseur selon la revendication 1, **caractérisé en ce que** les autres éléments extensibles (17) présentent, à l'état comprimé, une plus grande longueur dans la direction de l'axe longitudinal (a-a) de l'extenseur que les premiers éléments extensibles (13).

3. Extenseur selon la revendication 1, **caractérisé en ce que** les premiers éléments extensibles (13) et les autres éléments extensibles (17) sont en forme de losange à l'état comprimé, les autres éléments extensibles (17) étant reliés, en deux angles (17a, 17b) opposés approximativement dans la direction périphérique de l'extenseur, aux premiers éléments extensibles (13) qui sont reliés à leurs deux autres angles (13c, 13d) de leur forme en losange, aux éléments intermédiaires (16).

4. Extenseur selon la revendication 1, **caractérisé en ce qu'**un premier élément extensible (13) et un autre élément extensible (17) sont prévus alternativement le long du pourtour de l'extenseur.

5. Extenseur selon la revendication 1, **caractérisé en ce que** les angles et les bords des éléments extensibles (13, 17) et/ou des éléments intermédiaires (16) sont arrondis et optimisés sur le plan de l'écoulement.

6. Extenseur selon la revendication 1, **caractérisé en ce que** d'autres éléments extensibles (17') extérieurs, situés à l'extrémité (10a, 10b) de l'extenseur, sont plus courts dans la direction longitudinale de l'extenseur que d'autres éléments extensibles (17) situés à l'intérieur.

7. Extenseur selon la revendication 6, **caractérisé en ce que** la moitié extérieure (17e') située à l'extrémité, de l'autre élément extensible extérieur (17') est plus courte que sa moitié intérieure (17f).

8. Extenseur selon la revendication 1, **caractérisé en ce que** des premiers éléments extensibles extérieurs (13') situés à l'extrémité (10a, 10b) de l'extenseur, sont plus longs dans la direction longitudinale de l'extenseur que des premiers éléments extensibles (13) situés à l'intérieur.

9. Extenseur selon la revendication 8, **caractérisé en ce que** la moitié extérieure (13e') située à l'extrémité (10a, 10b) du premier élément extensible extérieur (13') est plus longue que sa moitié intérieure (13f).

10. Extenseur selon la revendication 7, **caractérisé en ce que** des moitiés extérieures (13e'), situées à l'extrémité (10a, 10b) de l'extenseur, de premiers éléments extensibles extérieurs (13'), correspondent dans leur forme aux moitiés extérieures (17') des autres éléments extensibles extérieurs (17').

11. Extenseur selon la revendication 1, **caractérisé en ce que** des points de liaison (18, 19, 20) entre les éléments extensibles (13, 17) sont régulièrement espacés dans la direction périphérique.
